# EUROPÄISCHE PATENTSCHRIFT

(11) **EP 1 663 252 B1**
(45) Veröffentlichungstag und Bekanntmachung des Hinweises auf die Patenterteilung: **22.08.2012**
(21) Anmeldenummer: 04786930.0
(22) Anmeldetag: 10.09.2004
(51) Int. Cl.: A61K 31/60, A61K 9/06, A61K 9/08, A61M 35/00

(54) **APPLIKATIONSANORDNUNG FÜR AUF DIE HAUT AUFZUTRAGENDE HEILMITTEL UND VERWENDUNG EINER VERPACKUNG ALS APPLIKATIONSANORDNUNG**
APPLICATION ASSEMBLY FOR MEDICAMENTS THAT ARE TO BE APPLIED TO THE SKIN AND PACKAGING THAT IS USED AS AN APPLICATION ASSEMBLY
DISPOSITIF POUR APPLIQUER DES MEDICAMENTS SUR LA PEAU ET UTILISATION D'UN EMBALLAGE COMME DISPOSITIF D'APPLICATION

(30) Priorität: 10.09.2003 DE 10342095
(43) Veröffentlichungstag der Anmeldung: 07.06.2006
(73) Patentinhaber: P & M Cosmetics GmbH & Co. KG, 48291 Telgte (DE)
(72) Erfinder: ISERMANN, Detlef, 48291 Telgte (DE)
(74) Vertreter: Hoffmeister, Helmut
(86) Internationale Anmeldenummer: PCT/EP2004/010128
(87) Internationale Veröffentlichungsnummer: WO 2005/023268

(56) Entgegenhaltungen:
- WO-A-97/06076
- WO-A-99/04893

## Beschreibung

Die Erfindung betrifft die Verwendung einer Verpackung als galenisch geeignete Applikationsanordnung, wobei die Verpackung aus zwei verbundenen Behältern mit je einer Austrittsöffnung besteht, die beide in einem Mischraum enden, und wobei der Inhalt entsprechend den gewünschten Anteilen in den Mischraum einführbar ist, dort unmittelbar vor der Applikation mischbar und durch eine Spritzöffnung austragbar ist.

Es ist bekannt, direkt aus Tuben oder Behältern Mittel zur Behandlung der Haut auf die Haut als Tinktur oder Salbe aufzubringen. Es sind auch Behälter mit zwei Mischkammern bekannt, die es dem Verbraucher ermöglichen, individuell ein bestimmtes Kosmetikum oder Heilmittel für eine bestimmte Situation in unterschiedlicher Zusammensetzung aus einem Gebinde auszubringen. Aus der Beschreibung zu dem Deutschen Gebrauchsmuster 295 12 627 und der Schrift WO 97/06076 ist eine solche Vorrichtung bekannt. Aus der WO97/06076 A ist eine Vorrichtung zum Mischen und Ausbringen einer Mischsubstanz bekannt, wobei eine Kammer, die auch die Mischkammer sein kann, mit zumindest einer weiteren eine Substanz enthaltenden Kammer verbunden ist, und wobei die Mischsubstanz als Basis- oder Trägersubstanz und die Substanz als der Wirkstoff verstanden wird.

Für die Erfindung stellt sich die Aufgabe, bei einer speziellen Rezeptur, deren Bestandteile untereinander nicht beständig mischbar sind, die aber trotzdem auf die Haut zumindest für den Zeitraum des Auftragens in gemischtem Zustand aufgetragen werden sollen, die Verwendung einer Verpackung als galenisch geeignete Applikationsanordnung anzugeben. Bei der Rezeptur handelt es sich um ein topisch auf die Haut aufzutragendes tinkturartiges Heilmittel, das aus in einem organischen Lösungsmittel gelöster Salicylsäure und in Wasser gelöster Glycolsäure besteht. Dieses soll in geeigneter Weise angeboten und vorrätig gehalten werden können.

Diese Aufgabe wird durch den Anspruch1 gelöst.

Zur Behandlung von Akne ist die Anwendung von Salicylsäure-Lösungen bekannt (vergl. Zitat in EP 676 194 B1 und Hinweis dort auf DD 274 357). Auch die Verwendung von Glycolsäure-Lösungen für die Hautbehandlung ist bekannt (vergl. EP 676 194 B1). Bei gleichzeitiger Verwendung beider Substanzen ergibt sich eine synergistische Wirkung. Da jedoch Wasser und die meisten organischen Lösungsmittel, insbesondere Öle, praktisch nicht untereinander mischbar sind bzw. diese nicht in gemischtem Zustand aufbewahrt werden können, sind diese Lösungen getrennt aufzubewahren und aufzubringen.

WO99/04893 offenbart einen Behälter für wenigstens ein Körperpflegemittel, das einen Basismittelbereich und einen Wirkstoffbereich - beide voneinander getrennt - enthält. Beide Bereiche sind durch eine Mischkammer verbunden. Die hautverträgliche Basissubstanz kann eine wässrige Lösung, eine alkoholisch-wässrige Lösung neben Öl, Emulsion, Creme, Gelee, Milch, Gel sein. Der Wirkstoff stellt ein Sonnenschutzmittel dar, wie z.B. Salicylsäureester.

Der Anwender der Applikationsanordnung mit den genannten Substanzen wird in erster Linie ein Hautarzt sein. Ihm wird die Applikation hochkonzentrierter Salicylsäure mit einem niedrigen pH-Wert zusammen mit Glycolsäure erleichtert. Salicylsäure wird perkutan absorbiert, wobei bei

hohen Flächenkonzentrationen hier in einen nierenschädigenden Bereich eingetreten werden kann. Andererseits hat hochkonzentrierte Salicylsäure, etwa bis zu 20 Gewichts%, eine intensive hautabtragende (keratolytische) Wirkung, die erwünscht ist. Bei Verwendung der genannten Vorrichtung kann im vorliegenden Falle eine hohe Wirkung bei niedrigstem Risika erreicht werden, ohne dass vor der Anwendung der Arzt die Komponenten in einem Behälter vormischen muss.

Mit der Applikationsanordnung gemäß Erfindung ergibt sich damit eine gute Möglichkeit, beide Substanzen im gemischten Zustand topisch aufzubringen, in geeigneter Weise zu dosieren und aufzubewahren.

Die zu verwendenden Lösungen an sich, nämlich die in organischer Lösung vorhandene Salicylsäure und die in Wasser gelöste Glycolsäure, sind in ihren für bestimmte Anwendungsfälle erforderlichen Konzentrationen und in ihrer Zusammensetzung bekannt. Insbesondere soll die Glycolsäure-Lösung so eingestellt werden, dass Akne-befallene Hautpartien schichtweise abgetragen werden können, was durch das sogenannte Peeling geschieht, so dass anschlie-βend die Salicylsäure-Lösung besser einwirken kann.

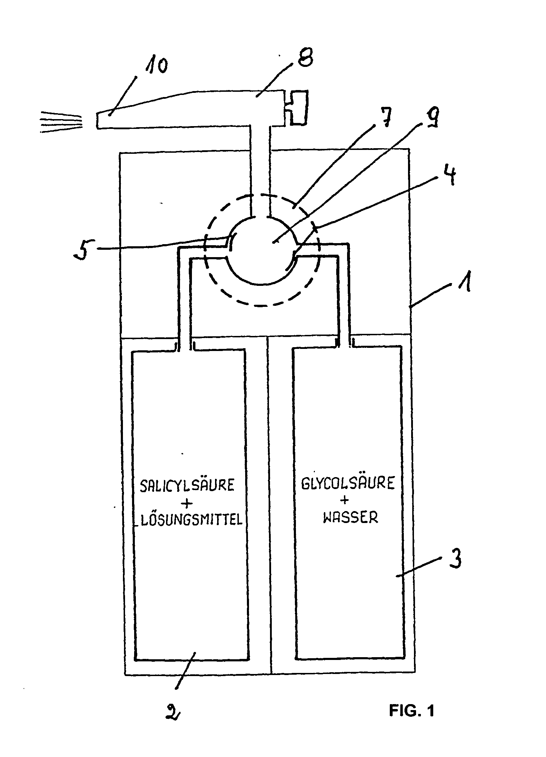

Eine Vorrichtung als Ausführungsbeispiel der Erfindung ist in der Zeichnung dargestellt.

In einem aus Kunststoff bestehenden Gehäuse 1 befinden sich zwei getrennte Behälter 2 und 3, nämlich ein Behälter 2 für Salicylsäure in einem geeigneten organischen Lösungsmittel und ein Behälter 3 für eine Glycolsäure-Wasser-Lösung. Beide Behälter sind mit Kanülen 2' bzw. 3' versehen, die in einem gemeinsamen Mischraum 9 münden.

Über Skalenringe 7 lassen sich zwei Schieber 4 und 5 getrennt verstellen, mit denen die Kanülen 2'und 3' teilweise verschlossen werden können. Mit Hilfe einer Kolbenpumpe 8 wird zunächst gleichzeitig anteilig der jeweilige Anteil an Salicylsäure-Lösung bzw. Glycolsäure-Lösung in den Mischraum 9 eingeführt und durch das Einführen gemischt. Zusätzliche Mischungsvorrichtungen, wie beispielsweise Mischkolben oder dergleichen, können vorgesehen werden. Über die Kolbenpumpe 8 wird dann der gemischte Inhalt der Mischkammer angehoben und über eine Düse 10 auf bestimmte Hautbereiche, das heißt topisch, als Tinktur aufgebracht.

Der obere Teil des Gehäuses 1 ist dabei durch entsprechende Fenster (nicht dargestellt) einsehbar gemacht, so dass die Funktion der Applikationsanordnung auch überprüft werden kann.

Als Materialien eignen sich Kunststoffe, die gegen die verwendeten Lösungsmittel unempfindlich sind. Es lassen sich aber auch Metallbehälter einsetzen. Weiterhin können Druckkolben oder dergleichen auch im Bezug auf die verwendeten Behälter 2 und 3 eingesetzt werden, um deren Inhalt über die Kanülen 2'und 3' einzuführen.

## Patentansprüche

1. Verwendung einer Verpackung als galenisch geeignete Applikationsanordnung zweier nicht oder nur schlecht auf Vorrat mischbarer Bestandteile, wobei die Verpackung aus zwei verbundenen Behältern (2,3) mit je einer Austrittsöffnung besteht, die beide in einem Mischraum (9) enden, und der Inhalt der Behälter entsprechend den gewünschten Anteilen in den Mischraum einführbar ist, dort unmittelbar vor der Applikation mischbar und durch eine Spritzöffnung (10) austragbar ist, so dass das Gemisch topisch als Heilmittel auf die Haut auftragbar ist, wobei einer der Behälter (2) der Applikationsanordnung mit in einem organischen Lösungsmittel gelöster hochkonzentrierter keratolytisch wirkender Salicylsäure mit hiedrigen pH-wet und der andere Behälter (3) mit in Wasser gelöster Glycolsäure gefüllt ist.

2. Verwendung einer Verpackung nach Anspruch 1, **dadurch gekennzeichnet, dass** die Konzentration der Salicylsäure den Wert von 20 Gew.-% nicht überschreitet.

3. Verwendung einer Verpackung nach Anspruch 1, **dadurch gekennzeichnet, dass** in den Mischraum (9) der Applikationsanordnung zwei Kanülen (2', 3') münden, die mit über Skalenringe (7) getrennt verstellbaren Schiebern (4,5) wenigstens teilweise verschließbar sind.

## Claims

1. Use of a package as an application assembly, which is suitable for dispensing galenical preparations, of two components which cannot be mixed or cannot be mixed very well in storage, wherein the package consists of two connected containers (2, 3) each with an outlet opening terminating in each case in a mixing chamber (9), and the content of the containers can be introduced in accordance with the desired proportions into the mixing chamber where they can be mixed directly prior to application and can be delivered through a spray opening (10) so that the mixture can be applied topically to the skin as a medicament, wherein one of the containers (2) of the application assembly is filled with highly concentrated, keratolytic salicylic acid which is dissolved in an organic solvent and has a low pH value, and the other container (3) is filled with glycolic acid which is dissolved in water.

2. Use of a package as claimed in claim 1, **characterised in that** the concentration of the salicylic acid does not exceed the value of 20 wt.%.

3. Use of a package as claimed in claim 1, **characterised in that** two cannulas (2', 3') issue into the mixing chamber (9) of the application assembly and can be closed at least partially by means of slides (4, 5) which are separately adjustable by means of graduated collars (7).

## Revendications

1. Utilisation d'un emballage comme dispositif galénique permettant l'application de deux composants non ou difficilement miscibles à l'avance, dans laquelle
- l'emballage est constitué de deux récipients (2,3), chacun doté d'un orifice de sortie débouchant dans une chambre de mélange (9),
- le contenu des récipients peut être introduit selon les proportions souhaitées dans ladite chambre pour y être mélangé juste avant l'application puis éjecté à travers un orifice de pulvérisation (10), de sorte telle que l'on peut appliquer le mélange de façon topique sur la peau, en tant que médicament,
- l'un des récipients (2) du dispositif d'application est rempli d'un acide salicylique très concentré à effet kératolytique, de faible pH, dissout dans un solvant organique, l'autre récipient (3) étant rempli d'acide glycolique dissout dans de l'eau.

2. Utilisation d'un emballage selon la revendication 1, **caractérisée en ce que** la concentration de l'acide salicylique ne dépasse pas un pourcentage en poids de 20.

3. Utilisation d'un emballage selon la revendication 1, **caractérisée par** la présence de deux canules (2', 3') débouchant dans la chambre de mélange (9) du dispositif d'application, lesdites canules étant au moins partiellement obturables au moyen de coulisseaux (4, 5) réglables séparément par le biais d'anneaux gradués (7).
